# EUROPEAN PATENT SPECIFICATION

(11) **EP 0 903 160 B1**
(45) Date of publication and mention of the grant of the patent: **05.03.2003**
(21) Application number: 98112503.2
(22) Date of filing: 06.07.1998
(51) Int. Cl.: A61M 16/08

(54) **Inspiratory tube**
Beatmungstubus
Tube respiratoire

(30) Priority: 11.09.1997 SE 9703291
(43) Date of publication of application: 24.03.1999
(73) Proprietor: Siemens-Elema AB, 171 95 Solna (SE)
(72) Inventor: Psaros, Georgios, 14638 Tullinge (SE)

(56) References cited:
- WO-A1-95/06234
- FR-A1- 2 724 322
- US-A- 5 111 827

## Description

The present invention relates to an inspiratory tube according to the preamble to claim 1.

One such inspiratory tube, connected to a ventilator, is described in WO 94/06499. The ventilator comprises an inspiratory tube in the form of an inspiratory limb, connected to a source of gas at one end and to the patient at the other end. At a specific distance from the patient, an expiratory valve is connected to the inspiratory limb. A pressure gauge and a flow meter are arranged in the inspiratory limb, between the source of gas and the expiratory valve, to measure pressure and flow in same. A control unit controls the entire ventilator. The known ventilator is mainly intended for use as a home care ventilator, i.e. a respirator a patient can use at home.

Interest in home care ventilators is steadily increasing. This is because the device is advantageous to the patient, who is able to be at home and can enjoy a greater degree of mobility. There are also public health advantages, since it would free up hospital resources by reducing in-patient treatment time, beds in intensive care being particularly expensive. This type of ventilator can be battery-powered and is then sometimes referred to as a 'portable' ventilator. This means that they are usually so heavy that they can only be carried around with some difficulty, even by healthy people.

A fully portable ventilator, i.e. a ventilator (weighing up to 1 kg and preferably less than 500 g) the patient is easily able to carry around, with a capacity sufficient to provide respiratory assistance for several hours would be even more desirable for patients. Miniaturising a ventilator to this extent, employing modern turbine, battery and microprocessor technology etc., is fully feasible. One important factor in this process would then be to minimise the number of parts to the greatest possible extent. An ability to receive all the required information on functions with no need for extensive additional equipment, such as extra tubing etc., would also be desirable. Such a fully portable ventilator would suit virtually every kind of patient. It could even be used as an emergency ventilator for a number of applications and be included in the basic equipment of e.g. aircraft, buses, boats, ambulances, fire engines etc.

The known inspiratory tube described above has limitations, since a plurality of connectors are needed for the pressure gauge and flow meter. The known inspiratory tube also has a design that limits its usefulness and makes necessary the use of separate gas sources or special valve systems for controlling the expiratory valve etc. connected to the inspiratory tube.

US 5,111,827 discloses an intubation tube with a connector. The connector includes a restriction and outflow tubes on either side of the restriction for measuring pressure and/or flow.

EP 366 524 discloses an intubation tube having channels within its wall for measuring pressure at the tip of the tube.

One object of the invention is to achieve an inspiratory tube that can be used in a simple and safe fashion for a plurality of functions with no need for special, additional tubing.

Another object of the invention is to achieve an inspiratory tube that is primarily suitable for miniaturisation into a fully portable ventilator.

These objects are achieved in accordance with the invention in that the inspiratory tube as described above comprises the features set forth in the characterising part of claim 1.

A number of advantages are achieved when the inspiratory valve is devised with a constriction in the gas flow channel, between one end of the inspiratory tube and an opening for an expiratory device, and channels that run inside the wall of the inspiratory tube and open onto either side of the constriction. The pressure gauge/flow meter can be connected to the channels and measure pressure/flow between the expiratory valve and the distal end of the inspiratory tube, i.e. close to the patient. These measurements are a prerequisite for a control unit to calculate and regulate a flow of gas in such a way that the flow of gas maintains a PEEP for the patient when the tube is used with a ventilator.

The ability to locate pressure/flow measurement facilities near the patient means that every attempt by the patient to take a breath can be sensed in a simple and reliable fashion. A new inhalation (inspiration) by the patient can then be quickly supported, since there is already a flow of breathing gas at the expiratory valve. So an inspiratory flow of gas at a specific pressure and with a specific flow profile can be quickly generated and delivered to the patient when this valve is closed. This rapid response to an attempt at inspiration is not available with the described, known inspiratory tube and ventilator.

Additional advantageous embodiments of the inspiratory tube will be evident from the dependent claims to claim 1.

The inspiratory tube can therefore be devised with a dispensing channel arranged in its wall, essentially parallel to the gas flow channel, and the dispensing channel is arranged with an opening into the gas flow channel between the opening and the distal end of the inspiratory tube. The dispensing channel can also be connected to a dispensing unit for dispensing an additive gas into the gas flow channel.

In the corresponding manner, an additional channel, from which gas samples can be extracted, can be arranged in the wall of the inspiratory channel. This channel can appropriately open into the gas flow channel between the opening and the distal end.

The constriction can appropriately be devised as an integral part of the inspiratory tube.

An expiratory control channel can also be arranged in the wall, essentially parallel to the gas flow channel, and devised with an opening into atmosphere between the opening and the inspiratory tube's proximal end. The expiratory control channel can be connected to an expiratory valve and a source of compressed gas for controlling expiratory phases.

The different channels run appropriately through one or more reinforcements of the wall of the inspiratory tube. These reinforcements can be devised so the cross-section of the inspiratory tube is unique and so the tube can only be connected to the ventilator in one way.

One embodiment of the inspiratory tube according to the invention will be described below in greater detail, referring to the following figures:
FIG. 1 shows the use of an inspiratory tube according to the invention in a fully portable ventilator;
FIG. 2 shows part of the inspiratory tube;
FIG. 3 shows a cross-section of the inspiratory tube;
FIG. 4 shows an embodiment of a ventilator with a connected inspiratory tube; and
FIG. 5 shows the inspiratory tube according to FIG. 2 with a connected expiratory device.

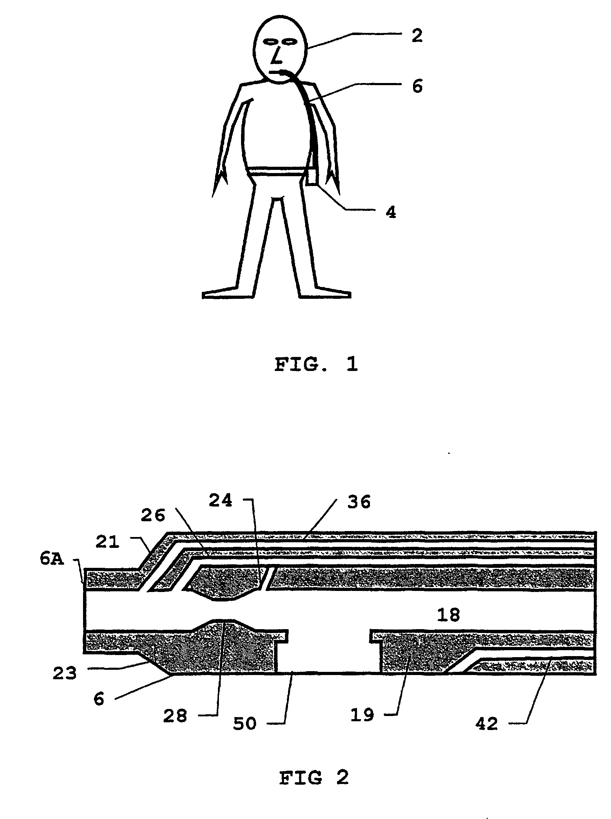

FIG. 1 shows how a patient 2 can become almost completely mobile with the aid of a fully portable ventilator 4. The patient 2 is connected to the ventilator 4 via an inspiratory tube 6 in an appropriate manner. A connection from the inspiratory tube 6 to the patient 2 via the nose is preferable with the fully portable ventilator 4, since the patient 2 would then find it easier to speak and communicate with others. It should be noted that the inspiratory tube 6 can also be attached in other ways, e.g. via a face mask, tracheal tube or tracheostomy/tracheotomy tube. The latter options are preferable when the ventilator is used for emergency cases or with patients 2 in need of stronger breathing support.

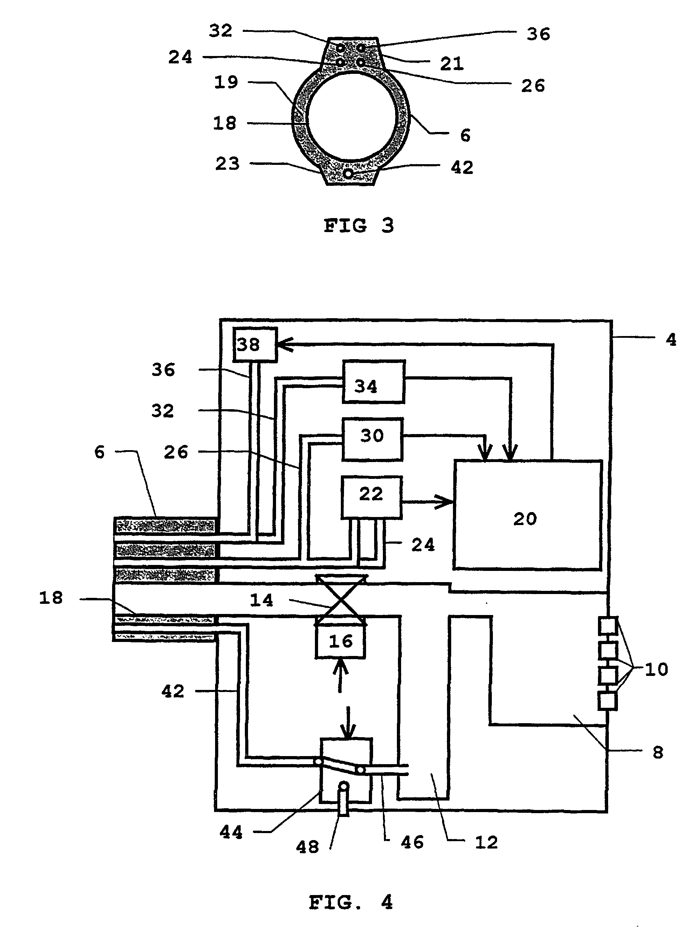

FIGS. 2 and 3 show details of the inspiratory tube 6. It should be noted that the inspiratory tube 6 could consist of a complete inspiratory line or be a part of an inspiratory line in a ventilator system. The design of the inspiratory tube 6 makes it particularly suitable for portable ventilator systems.

The inspiratory tube 6 according to the invention comprises a gas flow channel 18 surrounded by a casing or wall 19. An opening 50 is arranged at a specific distance from the distal end 6A of the inspiratory tube 6. An expiratory device (described below) can be connected to this opening 50. A constriction 28 is arranged in the gas flow channel 18 between the opening 50 and the distal end 6A. The constriction 28 causes a drop in the pressure of passing gas, the drop being proportional to the rate of flow. This accordingly makes it possible to measure both flow and pressure near the distal end 6A (to which a patient is attached when the inspiratory tube 6 is used) in a simple, space-saving fashion.

The wall 19 is devised with reinforcements 21, 23 on the upper and lower sides of the inspiratory tube 6 to provide space for a plurality of channels. These reinforcements 21, 23 (and distribution of the channels around the gas flow channel 18) can naturally be devised in virtually any fashion but are advantageously asymmetrical or arranged in such a way that the inspiratory tube's 6 cross-section is unique. This design then functions as key-indexing to guarantee correct connection of the inspiratory tube 6 to a ventilator or some other device intended for the inspiratory tube 6.

Thus, a first channel 24 runs through the upper reinforcement. The first channel 24 opens into the gas flow channel 18 between the constriction 28 and the opening 50. The first channel 24 can be connected at the other end to a pressure gauge (see below). A second channel 26 runs parallel to the first channel 24 through the upper reinforcement. The second channel 26 opens into the gas flow channel 18 between the constriction 28 and the distal end 6A. The second channel 26 can also be connected at the other end to a pressure gauge (see below).

A gas sampling channel 32 and a dispensing channel 36, both of which opening into the gas flow channel 18 between the constriction 28 and the distal end 6A, also run through the upper wall 19 reinforcement. An expiratory control channel 42 runs through the lower reinforcement of the wall 19. This channel opens into atmosphere at a point proximal to the opening 50. The objective of these channels 32, 36, 42 will be evident from the following.

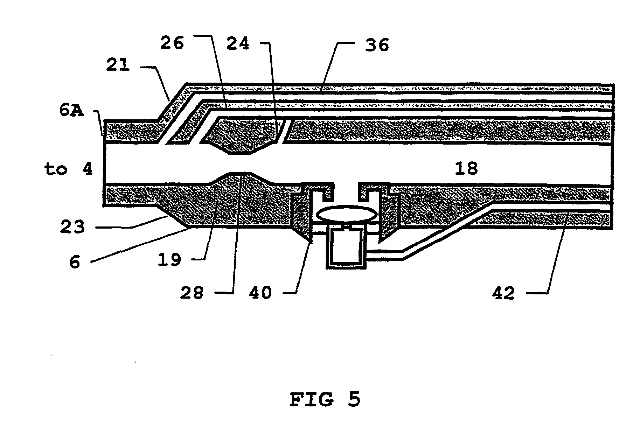

One embodiment of a ventilator 4, comprising an inspiratory tube 6, will now be described, referring to FIGS. 4 and 5. Gas pressure and flow are generated by a turbine 8 connected to atmosphere in some suitable fashion, e.g. via a number of openings 10 etc. These openings 10 can be equipped with filters to keep particles in breathing gas from being carried into the lungs. The turbine 8 is connected to a pressure tank 12 holding breathing gas, generated by the turbine 8, at a specific pressure. The pressure tank 12 is included so a desired flow of gas can be supplied to a patient more rapidly than would be the case if the turbine 8 had to generate flow and pressure from scratch. The pressure tank 12 is relatively small so it takes up less space and so the rise time for a pressure increase is faster with a smaller volume. A control valve 14 is arranged after the pressure tank 12. In this instance, the control valve 14 consists of a scissor valve controlled by a stepper motor 16. The control valve 14 controls the flow of gas out of the pressure tank 12 into the gas flow channel 18 and into the inspiratory tube 6. The control valve 14 is also small, enabling it to respond more rapidly to control signals (low inertia). This means, in turn, that breathing gas with steep gas pressure flanks can be delivered via the control valve 14. The gas flow channel 18 carries the breathing gas to the patient 4 at the distal end 6A of the inspiratory tube 6.

The turbine 8, pressure tank 12, control valve 14 and stepper motor 16 jointly constitute a gas flow generator capable of generating an optional flow of gas within the flow rate and pressure ranges relevant in the treatment of different kinds of patients. Even if the cited composition of the gas flow generator is viewed as advantageous, particularly in achieving fully portable ventilators 4, the gas flow generator can contain other constituents. For example, a compressor or a fan could replace the turbine 8. The turbine 8 could also be replaced by a source of pressurised gas, such as a gas cylinder or piped compressed air system. The pressure tank 12 can be rigid, distensible or omitted entirely. A variable-volume pressure tank could contribute to the generation of higher pressures by being compressible. In principle, the control valve 14 could consist of any known valve. The stepper motor 16 could be omitted or replaced with some other known actuator, depending on the control valve 14 selected. The composition of the gas flow generator affects the size and mobility of the ventilator 4. However, the same functionality is achieved irrespective of the composition. So the exact composition of the gas flow generator is not relevant to use of the invention.

The turbine 8 and stepper motor 16 are controlled by a control unit 20. Here, the control unit 20 can comprise hardware or software or any combination thereof. The control unit 20 controls all functions in the ventilator 4 in order to maintain the parameters set (via a user interface not shown). In order to maintain the functions, the control unit 20 must receive information on pressure and flow rate. The flow rate is obtained from a flow meter 22 which, via the first channel 24 and the second channel 26, is connected to the distal part of the inspiratory tube 6. In principle, the channels 24, 26 run parallel to the gas flow channel 18 inside the wall 19 of the inspiratory tube 6 and open, as described above, into the gas flow channel 18 on either side of the constriction 28. There is a drop in the pressure of gas flowing through the constriction 28, the drop being related to the rate of flow. Flow can accordingly be determined by the flow meter 22 when pressure is measured on either side of the constriction 28. Since pressure is utilised in this instance for determining the rate of flow, the second channel 26 can be connected to a pressure gauge 30 to measure the pressure at the distal end 6A of the inspiratory tube 6.

It should be noted that other flow meters, suitable for location in the vicinity of the distal end 6A, could replace the flow meter 22. A separate channel is then necessary for the pressure gauge 30 in the inspiratory tube 6. Since the flow meter 22 determines the flow rate from the pressure drop across the constriction 28, the pressure signal can be obtained straight from the flow meter 22 instead of from a separate pressure gauge 30.

The gas sampling channel 32, which opens into the inspiratory tube 6 at the distal end 6A, also runs through the wall 19 of the inspiratory tube 6. Gas samples can be extracted from the gas sampling channel 32 and analysed in a gas analysis unit 34. The gas analysis unit 34 appropriately comprises a pump for extracting the gas specimens to be analysed. The gas analysis can be performed to monitor carbon dioxide levels or to ensure that adequate breathing support is being provided. Gas analysis can also be performed in order to check the composition of the breathing gas supplied to the patient 4. If sufficiently fast gas and flow meters are used, carbon dioxide output and oxygen consumption can also be determined.

The latter is of interest when the inspiratory tube 6 has a dispensing channel 36 for dispensing an additive substance to the patient 4. The dispensing channel 36 is connected to a dispensing unit 38 and opens into the gas flow channel 18 at the distal end 6A. The dispensing unit 38 appropriately comprises a small reservoir to hold the substance and appropriate driving means for dispensing it through the dispensing channel 36. If the additive substance is a gas, the reservoir would appropriately consist of a gas cylinder containing the additive gas and a dispensing valve for regulating dispensing. The additive gas could consist of oxygen or some other gas, such as NO. The dispensing unit 38 could also contain medication or an additive, in liquid or powder form, which is dispensed through the dispensing channel 36 into the gas flow channel 18. The additive can be vaporised or atomised into small droplets (or grains of powder) and then supplied to the patient 4 in the breathing gas.

An expiratory valve 40 is also connected (in the opening 50 according to FIG. 2) to the inspiratory tube 6. In the illustrated embodiment, the expiratory valve 40 is fully incorporated into the wall 19 of the inspiratory tube 6 so it takes up as little space as possible. A tube or the like can also be connected between the inspiratory tube 6 and the expiratory valve 40 without affecting its function (as described below).

The expiratory valve 40 is connected to the gas flow channel 18 in such a way that the constriction 28 and the orifices of the channels 24, 26, 32, 36 lie between the expiratory valve 40 and the distal end 6A.

In this embodiment, the expiratory valve 40 is a pneumatically controlled ON/OFF valve (e. G a mushroom valve). It is connected to the pressure tank 12 via an expiratory control channel 42 and a switching valve 44. The expiratory control channel 42 runs inside the wall 19 of the inspiratory tube 6, parallel to the gas flow channel 18 and other channels 24, 26, 32, 36. The switching valve 44 is controlled by the control unit 20.

During inspiratory phases (inhalation phases), the switching valve 44 is in a first position in which the control channel 42 is connected to the pressure tank 12 via a first gas connection 46. The expiratory valve 44 is then closed with the same actuating pressure (usually higher than the pressure of the breathing gas delivered to the patient 4) as the pressure in the pressure tank 12.

During expiratory phases, the switching valve 44 is switched to a second position in which the expiratory valve 40 is connected to atmosphere via the control channel 42, the switching valve 44 and a second gas connection 48.

If e.g. a positive end-expiratory pressure (PEEP) is to be maintained for the patient 4, the control valve 14 is regulated in such a way that a flow of gas is released through the gas flow channel 18 towards the patient 4, even during expiration. This flow of gas is controlled by the control unit 20 according to the pressure and flow rate measured between the expiratory valve 40 and the distal end 6A. This regulated flow of gas also flows out through the expiratory valve 40 but simultaneously serves as resistance in relation to the patient 4 who accordingly exhales against a pressure corresponding to the selected PEEP.

This kind of PEEP regulation was not previously possible, especially not in portable ventilators. The design of the inspiratory tube 6, making it possible to locate pressure/flow measurement facilities between the expiratory valve 40 and the distal end 6A (the patient 4), plays a decisive role. PEEP cannot be regulated and maintained in this way unless information is available on pressure/flow at the patient 4.

This location for pressure/flow measurement also conveys other advantages. For example, the ventilator 4 can respond more rapidly to any efforts by the patient 4 to inhale (i.e. triggering). The ventilator 4 can, in particular, immediately respond to any inspiration commencing during expiration. Flow will then be registered as moving towards the patient 4, and the control unit 20 can respond immediately, closing the expiratory valve 40 and introducing an inspiratory flow of gas. This flow starts relatively quickly, since a flow of gas is already being maintained in the gas flow channel 18.

The placement of just about all the components in a common housing, with all the necessary gas channels arranged in the inspiratory tube 6, makes the ventilator 4 very compact and easy to use. The few parts (the housing and inspiratory tube 6) are easily interconnected in some suitable fashion. For example, known types of bayonet or pin index connectors can be used. No additional cords or tubing, which could become entangled with each other or other objects, are needed.

As noted above, the described embodiment is one very advantageous version of an inspiratory tube 6 for a fully portable ventilator that can be utilised for a number of different applications. For example, it can be used as a home ventilator for patients who do not require constant monitoring. It can also be used in ambulances or as an emergency ventilator. A third option is to use it for subacute treatment in hospitals. In other words, it can be employed for virtually every application for which a ventilator is needed. In all these instances, use is facilitated by the design of the inspiratory tube 6.

A portable ventilator is naturally battery-powered. The batteries can be rechargeable, and provision for parallel connection to an AC power source is an obvious option.

The ventilator does not need to incorporate all of the above options. The inspiratory tube 6 can be devised for both simpler and more complex versions of the ventilator 4. In principle, a simple version (of the ventilator 4) could comprise a turbine 8, pressure tank 12, control valve 14, stepper motor 16, control unit 20, flow meter 22 (doubling as a pressure gauge), inspiratory tube 6 (only with the channels 18, 24, 26 and 42), expiratory valve 40, switching valve 44 and batteries (not shown). This kind of simple version of the ventilator 4, utilising existing components, could be manufactured in about the same size as a portable cassette player or CD player, i.e. about 10*10*2 cm and weigh a hundred grams or so.

## Claims

1. An inspiratory tube (6) intended to serve at least as part of an inspiratory tube in a ventilator (4), comprising a wall (19), a distal end (6A), a proximal end, a gas flow channel (18) for carrying a flow of breathing gas and an opening (50) in the wall (19) at a distance from the distal end (6A) of the inspiratory tube (6), said opening (50) being devised for connection of an expiratory device (40), **characterised in that** a constriction (28) is arranged in the gas flow channel (18) between the distal end (6A) of the inspiratory tube (6) and the opening (50), a first channel (26) runs inside the wall (19), essentially parallel to the gas flow channel (18), and opens into the gas flow channel (18) between the distal end (6A) of the inspiratory tube (6) and the constriction (28) and a second channel (24) runs inside the wall (19), essentially parallel to the gas flow channel (18), and opens into the gas flow channel (18) between the constriction (28) and the opening (50), the channels (24, 26) being connectable to a pressure gauge (22, 30) for determining the gas flow's rate of flow and pressure.

2. The inspiratory tube according to claim 1, **characterised in that** a dispensing channel (36) is arranged in the wall (19), essentially parallel to the gas flow channel (18), and the dispensing channel (36) is devised with an opening into the gas flow channel (18) between the opening (50) and the distal end (6A) of the inspiratory tube (6), the dispensing channel (36) being connectable to a dispensing unit for dispensing an additive gas into the gas flow channel (18).

3. The inspiratory tube according to claims 1 or 2, **characterised in that** a gas sampling channel (32) is arranged in the wall (19), essentially parallel to the gas flow channel (18), and the gas sampling channel (32) is devised with an opening into the gas flow channel (18) between the opening (50) and the distal end (6A) of the inspiratory tube (6), the gas sampling channel being connectable to a gas analyser (34) for analysing the gas in the gas flow channel (18).

4. The inspiratory tube according to any of claims 1-3, **characterised in that** an expiratory control channel (42) is arranged in the wall (19), essentially parallel to the gas flow channel (18), and the expiratory control channel (42) is devised with an opening into atmosphere between the opening (50) and the proximal end of the inspiratory tube (6), the expiratory control channel (42) being connectable to an expiratory valve (40) and to a source of compressed gas (12) for controlling expiratory phases.

5. The inspiratory tube according to any of claims 1-4, **characterised in that** the constriction (28) is an integral part of the wall (19) of the inspiratory tube (6).

6. The inspiratory tube according to any of the claims 1-5, **characterised in that** the wall (19) is devised with at least one reinforcement (21, 23) in which at least one channel (24, 26, 32, 36, 42) is arranged.

7. The inspiratory tube according to claim 6, **characterised in that** the reinforcement (21, 23) is devised in such a way that the cross-section of the inspiratory tube (6) is asymmetrical.

## Patentansprüche

1. Ein Beatmungstubus (6) vorgesehen, um zumindest als Teil eines Inspirationstubus in einem Ventilator (4) zu dienen, mit einer Wand (19), einem distalen Ende (6A), einem proximalen Ende, einem Gasflusskanal (18) zum Transportieren eines Atemgasflusses und einer Öffnung (50) in der Wand (19) in einem Abstand von dem distalen Ende (6A) des Inspirationstubus (6), wobei die Öffnung (50) für den Anschluss einer Exspirationsvorrichtung (40) ausgebildet ist, **dadurch gekennzeichnet, dass** in dem Gasflusskanal (18) zwischen dem distalen Ende (6A) des Inspirationstubus (6) und der Öffnung (50) eine Einschnürung (28) vorgesehen ist, ein erster Kanal (26) innerhalb der Wand (19) im wesentlichen parallel zu dem Gasflusskanal (18) verläuft und zwischen dem distalen Ende (6A) des Inspirationstubus (6) und der Einschnürung (28) in den Gasflusskanal (18) mündet und ein zweiter Kanal (24) innerhalb der Wand (19) im wesentlichen parallel zu dem Gasflusskanal (18) verläuft und zwischen der Einschnürung (28) und der Öffnung (50) in den Gasflusskanal (18) mündet, wobei die Kanäle (24, 26) zur Bestimmung des Flusses und Druckes des Gasflusses mit einem Druckmesser (22, 30) verbindbar sind.

2. Der Beatmungstubus nach Anspruch 1, **dadurch gekennzeichnet, dass** ein Dosierkanal (36) in der Wand im wesentlichen parallel zu dem Gasflusskanal (18) vorgesehen ist und der Dosierkanal (36) mit einer Öffnung in den Gasflusskanal (18) zwischen der Öffnung (50) und dem distalen Ende (6A) des Inspirationstubus (6) ausgebildet ist, wobei der Dosierkanal (36) mit einer Dosiereinheit zum Dosieren eines additiven Gases in den Gasflusskanal (18) hinein verbindbar ist.

3. Ein Inspirationstubus nach Anspruch 1 oder 2, **dadurch gekennzeichnet, dass** ein Gassamplingkanal (32) in der Wand (19) im wesentlichen parallel zu dem Gasflusskanal (18) angeordnet ist und der Gassamplingkanal (32) mit einer Öffnung in den Gasflusskanal (18) zwischen der Öffnung (50) und dem distalen Ende (6A) des Inspirationstubus (6) versehen ist, wobei der Gassamplingkanal zum Analysieren des Gases in dem Gasflusskanal (18) mit einem Gasanalysator (34) verbindbar ist.

4. Der Inspirationstubus nach einem der Ansprüche 1-3, **dadurch gekennzeichnet, dass** ein exspiratiorischer Steuerkanal (42) in der Wand (19) im wesentlichen parallel zu dem Gasflusskanal (18) angeordnet ist und der exspiratorische Steuerkanal (42) mit einer Öffnung in die Atmosphäre zwischen der Öffnung (50) und dem proximalen Ende des Inspirationstubus (6) ausgebildet ist, wobei der exspiratorische Steuerkanal (42) zur Steuerung der Exspirationsphasen mit einem exspiratorischen Ventil (40) und mit einer Quelle komprimierten Gases (12) verbindbar ist.

5. Der Inspirationstubus nach einem der Ansprüche 1-4, **dadurch gekennzeichnet, dass** die Einschnürung (28) ein integraler Teil der Wand (19) des Inspirationstubus (6) ist.

6. Der Inspirationstubus nach einem der Ansprüche 1-5, **dadurch gekennzeichnet, dass** die Wand (19) zumindest mit einer Verstärkung (21, 23), in der zumindest ein Kanal (24, 26, 32, 36, 42) angeordnet ist, ausgebildet ist.

7. Der Inspirationstubus nach Anspruch 6, **dadurch gekennzeichnet, dass** die Verstärkung (21, 23) derart ausgebildet ist, dass der Querschnitt des Inspirationstubus (6) asymmetrisch ist.

## Revendications

1. Tube (6) d'inspiration destiné à servir au moins en tant que partie d'un tube d'inspiration dans un ventilateur (4), comportant une paroi (19), une extrémité (6A) distale, une extrémité proximale, un canal (18) pour l'écoulement de gaz pour acheminer un écoulement de gaz de respiration et une ouverture (50) dans la paroi (19) à une distance de l'extrémité (6A) distale du tube (6) d'inspiration, l'ouverture (50) étant conçue pour une connexion d'un dispositif (40) d'expiration, **caractérisé en ce qu'**une restriction (28) est disposée dans le canal (18) d'écoulement gazeux entre l'extrémité (6A) distale du tube (6) d'inspiration et l'ouverture (50), un premier canal (26) s'étend à l'intérieur de la paroi (19), sensiblement parallèlement au canal (18) d'écoulement de gaz, et débouche dans le canal (18) d'écoulement de gaz entre l'extrémité (6A) distale du tube (6) d'inspiration et la restriction (28) et un deuxième canal (24) s'étend à l'intérieur de la paroi (19), sensiblement parallèlement au canal (18) d'écoulement de gaz, et débouche dans le canal (18) d'écoulement de gaz entre la restriction (28) et l'ouverture (50), les canaux (24, 26) pouvant être connectés à une jauge (22, 30) de pression pour déterminer le débit de l'écoulement gazeux et la pression.

2. Tube d'inspiration suivant la revendication 1, **caractérisé en ce qu'**un canal (36) de dispensation est disposé dans la paroi (19) sensiblement parallèlement au canal (18) d'écoulement gazeux, et le canal (36) de dispensation est conçu avec une ouverture dans le canal (18) d'écoulement gazeux entre l'ouverture (50) et l'extrémité (6A) distale du tube (6) d'inspiration, le canal (36) de dispensation pouvant être connecté à une unité de dispensation pour dispenser un gaz additif dans le canal (18) d'écoulement gazeux.

3. Tube d'inspiration suivant la revendication 1 ou 2, **caractérisé en ce qu'**un canal (32) d'échantillonage du gaz est disposé dans la paroi (19), sensiblement parallèlement au canal (18) d'écoulement gazeux et le canal (32) d'échantillonnage du gaz est conçu en ayant une ouverture dans le canal (18) d'écoulement gazeux entre l'ouverture (50) et l'extrémité (6A) distale du tube (6) d'inspiration, le canal d'échantillonnage du gaz pouvant être connecté à un analyseur (34) du gaz pour analyser le gaz dans le canal (18) d'écoulement gazeux.

4. Tube d'inspiration suivant l'une quelconque des revendications 1 à 3, **caractérisé en ce qu'**un canal (42) de commande d'expiration est disposé dans la paroi (19), sensiblement parallèlement au canal (18) d'écoulement gazeux, et le canal (42) de commande d'expiration est conçu en ayant une ouverture dans l'atmosphère entre l'ouverture (50) et l'extrémité proximale du tube (6) d'inspiration, le canal (42) de commande d'expiration pouvant être connecté à une vanne (40) d'expiration et à une source de gaz (12) comprimé pour commander des phases d'expiration.

5. Tube d'inspiration suivant l'une quelconque des revendications 1 à 4, **caractérisé en ce que** la restriction (28) est une partie d'une pièce de la paroi (19) du tube (6) d'inspiration.

6. Tube d'inspiration suivant l'une quelconque des revendications 1 à 5, **caractérisé en ce que** la paroi (19) est conçue en ayant au moins un renforcement (21, 23) dans lequel au moins un canal (24, 26, 32, 36, 42) est disposé.

7. Tube d'inspiration suivant la revendication 6, **caractérisé en ce que** le renforcement (21, 23) est conçu de telle manière que la section transversale du tube (6) d'inspiration n'est pas symétrique.
